# FASCICULE DE BREVET EUROPEEN

(11) **EP 0 972 511 B1**
(45) Date de publication et mention de la délivrance du brevet: **09.04.2003**
(21) Numéro de dépôt: 99401133.6
(22) Date de dépôt: 07.05.1999
(51) Int. Cl.: A61K 7/48

(54) **Association d'un rétinoide avec un dérivé d'histidine**
Mischung bestehend aus ein Retinoid und eine Histidinederivate
Association of a retinoid with a histidine derivative

(30) Priorité: 26.05.1998 FR 9806603
(43) Date de publication de la demande: 19.01.2000
(73) Titulaire: L'OREAL, 75008 Paris (FR)
(72) Inventeur: Boussouira, Boudiaf, 75020 Paris (FR)
(74) Mandataire: Renard, Emmanuelle

(56) Documents cités:
- EP-A- 0 500 332
- WO-A-90/06102
- DE-A- 4 328 871
- FR-A- 2 756 565
- US-A- 5 441 740
- HIRONOBU MURASE: "Antioxidant and Emulsifying Activity of N-(Long-chain-acyl)histidine and N-(Long-chain-acyl)carnosine" J.AGRIC. FOOD CHEM., vol. 41, 1993, pages 1601-1604, XP002095666
- VASIL' EV ET AL.: "Derivatives of Hemin. Ligand-Exchange Chromatography of Hemin and its derivatives. Simple Synthesis of Hemin Amides With Amino Acids" JOURNAL OF ORGANIC CHEMISTRY USSR (ENGL. TRANSL.), vol. 14, 1978, pages 786-793, XP002095667
- TSUNODA T, ET AL: "Stability of all-trans-retinol in cream" J. SOC. COSMET. CHEM., vol. 4, 1995, pages 191-198, XP002095668

## Description

La présente invention a pour objet de nouvelles compositions cosmétiques et/ou dermatologiques, en particulier des compositions destinées au soin de la peau et comprenant au moins un composé de la famille des rétinoïdes. Plus particulièrement, l'invention a pour objet des compositions stables comprenant au moins un composé de la famille des rétinoïdes et au moins un dérivé d'histidine.

Les compositions cosmétiques et/ou dermatologiques à base de rétinoïdes ont connu un important développement au cours de ces dernières années. Parmi les rétinoïdes, l'utilisation de l'acide rétinoïque dans des compositions pour le traitement de l'acné est bien connue.

Toutefois, il est apparu que d'autres dérivés de la famille des rétinoïdes présentent un intérêt, à la fois pour le traitement de l'acné, et également pour le soin de la peau, en particulier pour limiter, voire supprimer, les effets du vieillissement sur la peau : les rides, l'aspect parcheminé, le jaunissement, la perte d'élasticité, la rugosité, la sécheresse, l'apparition de tâches sont les manifestations habituelles du vieillissement de la peau. Ces manifestations sont d'autant plus accentuées que la peau a été fréquemment exposée au soleil ou est particulièrement sensible à l'exposition aux rayonnements UV.

Ainsi, les effets du vieillissement intrinsèque de la peau (lié à l'âge) et du photo-vieillissement (dû à l'exposition au soleil) peuvent se cumuler. Les manifestations du vieillissement apparaissent habituellement à un âge avancé, toutefois, leur prévention doit être entreprise dès le début de la vie d'adulte par des soins appropriés.

Le traitement de la peau par des dérivés de la famille des rétinoïdes fait partie de ces soins préventifs ou curatifs des manifestations du vieillissement que sont : les rides, la peau parcheminée, le jaunissement, la perte d'élasticité, la rugosité, la sécheresse, les tâches.

Parmi les dérivés de la famille des rétinoïdes, le rétinol, également connu sous le nom de vitamine A et les dérivés estérifiés du rétinol présentent un intérêt tout particulier. En effet, le rétinol est un constituant endogène naturel de l'organisme humain. Il est bien toléré en application sur la peau jusqu'à des taux beaucoup plus élevés que l'acide rétinoïque. Les esters du rétinol sont convertis en rétinol par l'organisme humain.

Toutefois, lorsqu'ils sont introduits dans une composition cosmétique ou dermatologique destinée à une application topique, le rétinol et ses esters se dégradent rapidement, sous l'effet de la lumière, de l'oxygène, des ions métalliques, des agents oxydants, de l'eau ou en particulier sous l'effet d'une élévation de température. La dégradation thermique du rétinol a fait l'objet d'une étude publiée dans J. Soc. Cosm. Chem. 46, 191 - 198 (July-August 1995).

On connaît différentes associations du rétinol ou d'autres dérivés de la famille des rétinoïdes avec des anti-oxydants, les rétinoïdes présentant une stabilité améliorée au sein de ces associations :

Le document WO93/00085 décrit des émulsions E/H comprenant du rétinol et un système stabilisant constitué d'un agent chélatant, comme par exemple l'EDTA, et un anti-oxydant qui peut être soit un anti-oxydant liposoluble, comme le butyl hydroxy toluène (BHT) ou la vitamine E, soit un anti-oxydant hydrosoluble, comme la vitamine C. Selon ce document, on peut également préparer des émulsion E/H contenant du rétinol stabilisé par un système constitué d'un anti-oxydant liposoluble et d'un anti-oxydant hydrosoluble.

Le document EP-608433 décrit des compositions contenant du rétinol et un agent stabilisant choisi parmi les agents chélatants et les polysaccharides, les huiles d'indice d'iode supérieur à 70, les polyéthylène (propylène) glycols, les sels d'acides hydroxycarboxyliques, les sels d'acides aminés neutres, les anti-oxydants liposolubles associés à l'EDTA et à une benzophénone, les anti-oxydants liposolubles associés à un composé acide et à une benzophénone, les dérivés de cyclodextrine dans lesquels est inclus un anti-oxydant ou un filtre UV, le butanediol et/ou les anti-oxydants liposolubles, les dérivés de benzophénone hydrosolubles, les acides aminés basiques et leurs sels, les acides aminés acides et leurs sels, les huiles polaires, les argiles minérales hydrophiles.

Enfin, on connaît de EP-500332 et de la publication "Antioxydant and Emulsifying Activity of N-(long-chain-acyl) histidine and N-(long-chain-acyl) carnosine", Hironobu Murase, J. Agric. Food Chem., Vol. 41, 1993, pp. 1601-1604 l'utilisation de dérivés N-acylés de camosine et d'histidine comme anti-oxydants, et en particulier comme inhibiteurs de la formation de peroxydes lipidiques, en présence desquels le rétinol est connu pour être instable.

Toutefois, aucun des composés de l'art antérieur, qu'il soit décrit comme anti-oxydant ou plus spécifiquement comme stabilisateur de rétinoïde, ne permet d'obtenir une stabilisation satisfaisante des rétinoïdes.

Or, la demanderesse a découvert avec étonnement que l'association d'un rétinoïde choisi parmi la vitamine A (ou rétinol) et les précurseurs bio-convertibles de la vitamine A, avec certains dérivés d'histidine, permettait d'éviter la dégradation, en particulier la dégradation thermique, de ces rétinoïdes. Ainsi, ces rétinoïdes peuvent être introduits dans des compositions cosmétiques et/ou dermatologiques, être stockés pendant plusieurs mois, sans voir se dégrader leur efficacité.

L'invention a donc pour objet l'association de :
- au moins un rétinoïde choisi parmi : la vitamine A (ou rétinol), le rétinal et les précurseurs bio-convertibles de la vitamine A,
et
- au moins un dérivé d'histidine répondant à la formule générale (I) suivante : 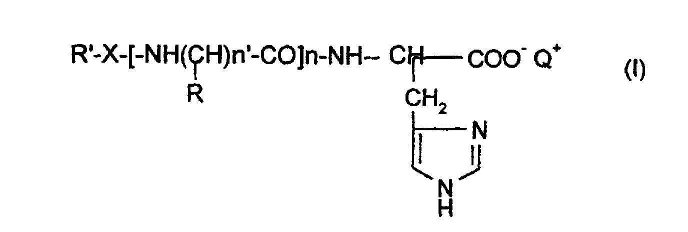
dans laquelle :
n est un entier allant de 0 à 5,
n' est un entier allant de 1 à 16,
R représente une chaîne latérale d'un acide aminé,
X représente un radical choisi parmi les radicaux de formules, lues de gauche à droite : -CO-, -O-CO-, -NH-CO-, -SO2-, -NH-CO-CO-, -O-CO-CO-,
R' représente un radical alkyle linéaire ou ramifié, saturé ou insaturé, éventuellement hydroxylé, en C6 à C22 ou un radical aminoalkyle en C6 à C22, la fonction amine étant événtuellement protégée sous forme d'acétamide ou substitué par un ou deux groupements alkyles inférieurs,
à la condition que R' soit un radical aminoalkyle tel que défini ci-dessus lorsque X = -CO-
Q⁺ représente H⁺ ou un cation organique ou minéral,
ainsi que les sels d'addition d'un composé de formule (I) avec un acide organique ou minéral.

Par vitamine A, on entend le rétinol de type all-trans ou de type 13-cis.

L'expression précurseur bio-convertible de la vitamine A désigne tout composé susceptible d'être converti en vitamine A par l'organisme humain. Parmi ces composés, on peut citer les esters de rétinol, en particulier les esters en C₁-C₆ qui sont très rapidement dégradés en rétinol par l'organisme humain. Parmi les esters du rétinol, on entend plus particulièrement l'acétate de rétinol et le propionate de rétinol.

L'invention a également pour objet une composition cosmétique ou dermatologique comprenant l'association telle que décrite ci-dessus et au moins un support physiologiquement acceptable.

L'invention a en outre pour objet l'utilisation d'au moins un dérivé d'histidine de formule (I), tel que défini ci-dessus, pour améliorer la stabilité d'une composition comprenant un rétinoïde choisi parmi la vitamine A (ou rétinol), le rétinal et les précurseurs bio-convertibles de la vitamine A.

L'invention a également pour objet un procédé pour améliorer la stabilité d'une composition comprenant un rétinoïde choisi parmi la vitamine A (ou rétinol), le rétinal et les précurseurs bio-convertibles de la vitamine A, ce procédé consistant à associer audit rétinoïde une quantité efficace d'au moins un dérivé d'histidine de formule (I), tel que défini ci-dessus.

Par quantité efficace de dérivé d'histidine de formule (I), on entend une quantité suffisante pour obtenir une amélioration notable et significative de la stabilité thermique du ou des rétinoïdes contenus dans la composition. Cette quantité minimale en agent stabilisant à mettre en oeuvre, qui peut varier selon la nature du support physiologiquement acceptable retenu pour la composition, peut être déterminée sans aucune difficulté au moyen d'un test de mesure de stabilité thermique, tel que celui donné dans les exemples ci-après.

Ainsi l'invention a encore pour objet l'utilisation de l'association d'au moins un rétinoïde choisi parmi la vitamine A (ou rétinol), le rétinal et les précurseurs bio-convertibles de la vitamine A et d'au moins un dérivé d'histidine de formule (I) dans ou pour la préparation d'une composition destinée au traitement dermatologique de lutte contre, et/ou de prévention de l'irritation, l'inflammation, l'immunosuppression et/ou l'acné.

L'invention a, en outre, pour objet un procédé de traitement cosmétique consistant à lutter contre les signes du viellissement, particulièrement les signes du vieillissement induits par la photoperoxydation, en particulier la photoperoxydation du squalène et/ou du collagène, par application topique sur la peau et/ou le cuir chevelu et/ou les cheveux d'une composition comprenant au moins un rétinoïde choisi parmi la vitamine A (ou rétinol), le rétinal et les précurseurs bio-convertibles de la vitamine A et au moins dérivé d'histidine de formule (I) selon l'invention. Elle a aussi pour objet l'utilisation d'une telle composition cosmétique pour lutter contre et/ou prévenir les signes du vieillissement photoinduits de la peau et/ou des cheveux. Elle a également pour objet une composition dermatologique comprenant une association selon l'invention destinée à lutter contre les signes du vieillissement cutané ou des cheveux, particulièrement les signes du vieillissement induits par la photoperoxydation, en particulier la photoperoxydation du squalène et/ou du collagène.

D'autres caractéristiques, aspects et avantages de la présente invention apparaîtront à la lecture de la description détaillée qui va suivre.

De préférence, le pH de l'association selon l'invention est supérieur ou égal à 6, de préférence supérieur ou égal à 7. Le pH de l'association selon l'invention est généralement inférieur ou égal à 12, de préférence inférieur ou égal à 10.

Le cation organique (Q⁺) des dérivés d'histidine de formule (I) peut être choisi parmi les ammoniums comportant un reste choisi parmi les aminoacides basiques tels que la lysine, l'arginine, ou bien encore parmi les amino-alcools tels que la glucamine, la N-méthyl glucamine, l'amino-3 propanediol-1,2.

Le cation minéral (Q⁺) peut être choisi parmi les sels alcalins ou alcalino-terreux tels que Na⁺, K⁺, ou peut être l'ion NH₄⁺.

Les sels d'addition avec un acide sont choisis par exemple parmi les chlorhydrates, les bromhydrates, les sulfates, les tartrates et les acétates.

Les composés de formule (I) comportent dans leur structure chimique au moins un carbone asymétrique : l'invention concerne aussi bien les composés à configuration D, à configuration L que leurs mélanges, en particulier le mélange racémique des composés D et L.

Selon l'invention, les composés préférés de formule (I) présentent au moins une des caractéristiques suivantes :
R' désigne de préférence un radical alkyle, linéaire ou ramifié, saturé, ayant de 8 à 18 atomes de carbone,
n est un entier de 1 à 5, et
n' est un entier allant de 1 à 11.

Selon la présente invention, parmi les radicaux alkyle linéaire ou ramifié ayant de 6 à 22 atomes de carbone, on peut citer avantageusement les radicaux hexyle, octyle, nonyle, 2-éthyl-hexyl, dodécyle, hexadécyle et octadécyle.

Les groupements alkyle inférieurs comprennent généralement de 1 à 6 atomes de carbone. On peut citer, comme groupement alkyle inférieur, les radicaux méthyle, éthyle, propyle, isopropyle, tertiobutyle, hexyle.

Parmi les radicaux alkyle linéaire ayant de 6 à 22 atomes de carbone, on peut citer notamment les radicaux octyle, dodécyle, hexadécyle et octadécyle.

Parmi les radicaux alkyle ramifiés ayant de 6 à 22 atomes de carbone, on peut citer notamment les radicaux 2-méthylpentyle, 1-méthylhexyle, 3-méthylheptyle.

Par radical alkyle insaturé, on préfère un radical ayant de 6 à 22 atomes de carbone linéaire ou ramifié comportant une ou plusieurs doubles liaisons.

Les chaînes latérales d'un acide aminé correspondent aux chaînes latérales de l'un quelconque des amino-acides naturels. Ainsi, R peut représenter notamment l'hydrogène, un radical méthyle ou isopropyle. Cela peut donc être des chaînes latérales non polaires, polaires mais non chargées, chargées négativement ou positivement.

Le procédé général de préparation des composés de formule (I) comprend l'étape qui consiste à faire réagir avec l'histidine dans un solvant inerte, un composé de formule (II) 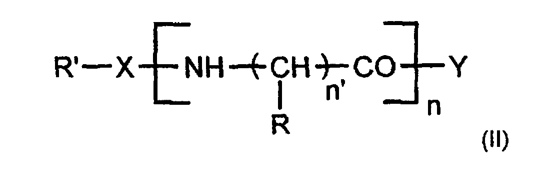 dans laquelle n', n, R, R' et X ont les mêmes significations que dans la formule (I) ci-dessus, Y est un groupement activant classique de la fonction acide.

Les réactions d'activation des groupements acides -COOH sont bien connues de l'homme du métier. On peut par exemple se reporter à "Advanced Organic Chemistry, Jerry March, 3^{ème} édition, 1985, p.370-377". On entend par agent de couplage tout composé susceptible de substituer le groupe OH du composé de formule (IV), puis d'être substitué ensuite par l'acide aminé que l'on souhaite greffer, par exemple l'histidine. Des agents de couplage sont cités dans "Advanced organic chemistry, J March, 3^{ème} édition, 1985, page 372. On peut notamment utiliser comme agent de couplage le 2-(5-norbornène-2,3-dicarboximido)-1,1,3,3 tétraméthyluronium tétrafluoroborate.

L'histidine de départ comportant un carbone asymétrique, est utilisée dans la forme optique, pure ou en mélange (D; L; D,L) selon la forme optique désirée du composé de formule (I).

Comme solvant, on peut utiliser le dichlorométhane, le dichloro-1,2 éthane, le trichloro-1,1,1 éthane, le chloroforme, l'acétonitrile, le toluène, le dioxane, le tétrahydrofurane, le diméthoxy-1,2 éthane, le cyclohexane, le diméthylformamide, l'eau ou un mélange de ces solvants.

La réaction est effectuée à une température comprise de préférence entre -10°C et + 40°C, et plus préférentiellement entre 20°C et 30°C.

La réaction peut être effectuée en présence d'une base. Celle-ci peut être choisie parmi les hydroxydes de métaux alcalins ou alcalino-terreux, l'hydrogénocarbonate de sodium, les alcoolates de métaux alcalins, les hydrures alcalins, les amines tertiaires telles que la pyridine, la di-isopropyl éthylamine ou la triéthylamine. On utilise de préférence l'hydrogénocarbonate de sodium.

Les composés préférés correspondant à la formule générale (1) sont ceux qui sont choisis parmi les formules suivantes : 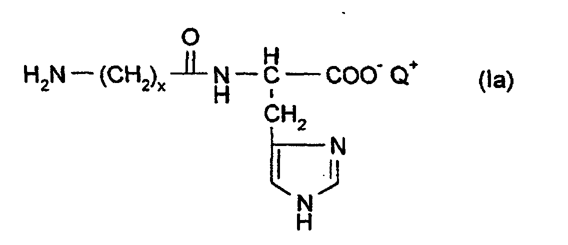 dans laquelle :
x est un entier allant de 6 à 22 et Q⁺ étant tel que décrit précédemment, et 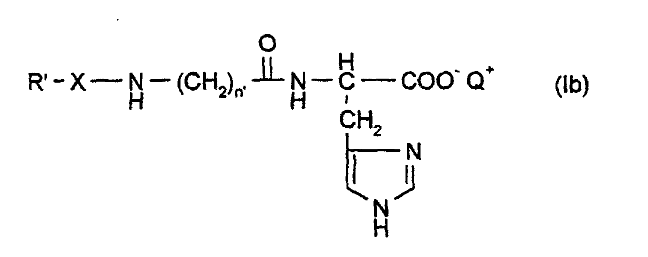
dans laquelle :
R', X, Q⁺ et n' sont tels que décrits précédemment.

Les composés de formule (la) sont tels que décrits dans la demande de brevet français n° 9803150 déposée le 13.03.1998 par la Demanderesse.

Les composés de formule (Ib) sont notamment tels que décrits dans la demande de brevet français n° 9614880 déposée le 04.12.1996 par la Demanderesse.

Les composés de formule (la) sont obtenus par un procédé qui consiste à faire réagir avec l'histidine dans un solvant inerte, un composé de formule (II)

R"NH-(CH₂)ₓ-COOH (II)

dans laquelle n a la même signification que dans la formule (I) ci-dessus, R" représente un groupe protecteur de la fonction amine, la fonction acide du composé (II) étant activée, par exemple en présence d'un agent de couplage.

Les composés de formule (Ib), lorsque X représente -O-CO-, sont obtenus par un procédé qui consiste à faire réagir dans un solvant inerte, un composé de formule (II) 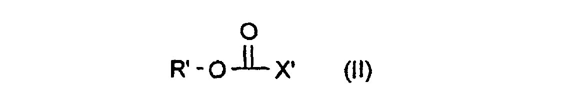 X' représentant un atome d'halogène, notamment un atome de chlore, ou un radical dérivé d'un azole, notamment un radical provenant d'un imidazole tel que celui de formule (III) : 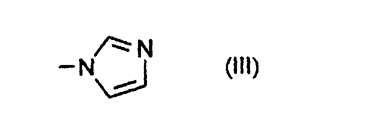 et R' ayant la même signification indiquée dans la formule (Ib) ci-dessus,
(A) soit avec la camosine, ou bien
(B) soit, dans une première étape, avec un amino-acide de formule :

   H₂N-(CH₂)_{n'}-COOH

   pour former un composé de formule (IV) suivante : 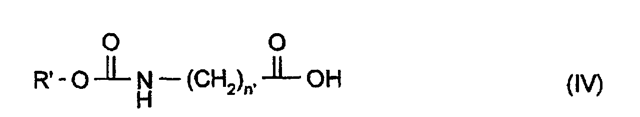
R' et n' ayant les mêmes significations que celles indiquées dans la formule (I) définie ci-dessus,
et, dans une deuxième étape, à faire réagir l'histidine avec le composé de formule (IV), en présence d'un agent de couplage.

Parmi les composés préférés correspondant à la formule générale (I), on peut notamment citer :
- la N-octyloxycarbonyl-β-alanyl-L-histidine (composé 1),
- la N-dodecyloxycarbonyl-β-alanyl-L-histidine (composé 2),
- la N-(12-amino-1-oxododécyl)-L-histidine (composé 3),
- le chlorhydrate de N-2-éthylhexyloxycarbonyl-β-alanyl-L-histidine,
- la N-hexadécyloxycarbonyl-β-alanyl-L-histidine,
- la N-octylaminocarbonyl-β-alanyl-L-histidine,
- la N-dodecylaminocarbonyl-β-alanyl-L-histidine,
- la N-dodecylsulfonyl-β-alanyl-L-histidine,
- la N-dodecylamino-oxalyl-β-alanyl-L-histidine.

L'homme du métier saura, par de simples essais, adapter la proportion relative de rétinoïde et de dérivés d'histidine de formule (I) pour obtenir l'effet recherché. En effet, les proportions optimales des différents constituants peuvent varier, par exemple en fonction du poids moléculaire du polymère, du taux d'amines et/ou du taux d'amines tertiaires dans ce polymère.

Outre le rétinoïde et le dérivé d'histidine de formule (I), l'association selon l'invention comprend avantageusement au moins un filtre solaire. L'adjonction d'un filtre solaire permet de renforcer la stabilité de l'association du rétinoïde avec le polymère polyaminé, en limitant l'action néfaste des UV sur le rétinoïde. Un tel constituant peut être choisi parmi les familles connues de filtres solaires actifs dans l'UVA et/ou l'UVB, hydrophiles ou lipophiles. On peut citer par exemple : les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3(4-méthylbenzylidène)camphre, les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxydibenzoylméthane, les dérivés de β,β-diphénylacrylate tels que le α-cyano-β,β-diphénylacrylate de 2-éthylhexyle, les dérivés de l'acide p-aminobenzoïque comme par exemple le paradiméthylaminobenzoate d'octyle, l'anthranilate de menthyle, les polymères filtres et silicones filtres décrits dans la demande WO-93-04665, les filtres hydrophiles contenant au moins un radical sulfonique -SO₃H, comme par exemple l'acide 2-phénylbenzimidazole 5-sulfonique ou l'acide benzène 1,4-di(3-méthylidène-10-camphosulfonique.

On peut également incorporer à l'association selon l'invention un autre composé connu de l'art antérieur comme stabilisateur du rétinol. Parmi ces composés on peut citer: les agents chélatants, et les polysaccharides, les huiles ayant un indice d'iode supérieur ou égal à 70, les polyéthylène glycol et/ou les polypropylène glycols, les hydroxycarboxylates, les acides aminés et leurs sels, les anti-oxydants tels que le butyl hydroxytoluène, le butyl hydroxyanisole, les α, β, γ, δ-tocophérols, la nordihydrogaiaretine, le gallate de propyle, les esters d'acides gras et de vitamine C, l'acide ascorbique, les sels d'acide ascorbique, l'acide iso-ascorbique, les sels d'acide iso-ascorbique, l'acide sorbique, les sels d'acide sorbique, le butanediol, les esters d'acide gras et de pentaérythritol, les esters gras de triméthylolpropane, les argiles minérales hydrophiles. De tels composés sont connus comme des stabilisants des rétinoïdes, on peut à ce sujet se reporter au document : EP-A-608433.

Dans le cas particulier du traitement de l'acné, on peut également incorporer dans l'association selon l'invention, de façon avantageuse, au moins un agent antiacnéïque spécifique, antiséborrhéique et/ou antibactérien, et en particulier, la piroctone olamine, commercialisée sous la dénomination Octopirox par la société HOECHST.

Dans les compositions selon l'invention, le rétinoïde est de préférence introduit en une quantité allant de 0,0001 à 10% en poids par rapport au poids total de la composition, avantageusement de 0,001 à 3%, encore plus préférentiellement de 0,01 à 1%.

Dans les compositions selon l'invention, le dérivé d'histidine de formule (I) est de préférence introduit en une quantité allant de 0,001 à 20% en poids par rapport au poids total de la composition, avantageusement de 0,05 à 10%.

Les compositions de l'invention peuvent comprendre en outre des adjuvants cosmétiques ou dermatologiques choisis parmi les corps gras, les solvants organiques, les émulsionnants, les épaississants non ioniques, les adoucissants, les antioxydants, les opacifiants, les stabilisants, les silicones, les agents anti-mousse, les agents hydratants, les vitamines, les parfums, les conservateurs, les tensioactifs, de préférence non ioniques, les charges, les séquestrants, les polymères autres que ceux décrits ci-dessus, les propulseurs, les agents alcalinisants ou acidifiants, les colorants, ou tout autre ingrédient habituellement utilisé en cosmétique.

Les corps gras peuvent être constitués par une huile ou une cire ou leur mélange, la vaseline, la paraffine, la lanoline, la lanoline hydrogénée, la lanoline acétylée ; ils comprennent également les acides gras, les alcools gras tels que l'alcool laurique, cétylique, myristique, stéarique, palmitique, oléique ainsi que le 2-octyldodécanol, les esters d'acides gras tels que le monostéarate de glycérol, le monostéarate de polyéthylèneglycol, le myristate d'isopropyle, l'adipate d'isopropyle, le palmitate d'isopropyle, le palmitate d'octyle, les benzoates d'alcools gras en C₁₂-C₁₅ (Finsolv TN de FINETEX), l'alcool myristique polyoxypropyléné à 3 moles d'oxyde de propylène (WITCONOL APM de WITCO), les triglycérides d'acides gras en C₆-C₁₈ tels que les triglycérides d'acide caprylique/caprique.

On peut citer parmi les huiles utilisables dans la présente invention, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide des huiles d'hydrocarbures telles que des huiles de paraffine, le squalane, la vaseline; des esters tels que le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, le triisostéarate de glycérine ; des huiles de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées ; des huiles perfluorées et/ou organofluorées ;des acides gras supérieurs tels que l'acide myristique, l'acide caprique, l'acide caprylique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique.

Les cires peuvent être choisies parmi les cires animales, végétales, minérales ou synthétiques. Parmi les cires animales, on peut notamment citer les cires d'abeilles, la cire de baleine. Parmi les cires végétales, on peut citer, entre autres, les cires de Camauba, de Candellila, d'Ouricoury, les cires de fibres de liège, les cires de canne à sucre et les cires du Japon. Parmi les cires minérales, on peut citer, en particulier, les cires de paraffine, la lanoline, les cires microcristallines, les cires de lignite et les ozokérites. Parmi les cires synthétiques, on peut citer, notamment, les cires de polyéthylène et les cires obtenues par synthèse de Fisher et Tropsch. Toutes ces cires sont bien connues de l'Homme de l'Art.

Parmi les solvants organiques, on peut citer les alcools et polyols inférieurs tels que l'éthanol, l'isopropanol, le propylèneglycol, la glycérine et le sorbitol.

Les épaississants, de préférence non ioniques, peuvent être choisis parmi les gommes de guar et celluloses modifiées ou non telles que la gomme de guar hydroxypropylée, la cétylhydroxyéthylcellulose, les silices comme par exemple la Bentone Gel MiO vendue par la société NL INDUSTRIES ou la Veegum Ultra, vendue par la société POLYPLASTIC.

Bien entendu, l'homme de l'art veillera à choisir ce ou ces éventuels composés complémentaires et/ou leurs quantités de manière telle que les propriétés avantageuses attachées intrinsèquement à la composition conforme à l'invention ne soient pas, ou substantiellement pas, altérées par la ou les adjonctions envisagées.

Les compositions selon l'invention peuvent se présenter sous la forme d'une lotion, d'un gel, d'une émulsion eau-dans-huile, d'une émulsion huile-dans-eau, d'une émulsion triple. Elles peuvent également se trouver sous une forme vectorisée, comme par exemple sous forme de nanocapsules, de liposomes, de nanoémulsions, d'oléosomes.

Ces compositions sont destinées à une application sur la peau du corps, en particulier la peau du visage et des mains, sur les muqueuses et les semi-muqueuses, ou encore sur les cheveux.

De telles compositions peuvent être sous la forme de produits de soin ou de maquillage. En particulier elles peuvent se présenter sous la forme d'une crème de soin, d'un lait, d'un tonique, d'un produit nettoyant et/ou démaquillant, d'un masque, d'un produit de gommage, d'un exfoliant, d'un produit de protection solaire, d'un fond de teint, d'une crème teintée, d'un rouge-à-lèvres.

D'autres caractéristiques et avantages de la composition de l'invention ressortiront mieux des exemples et contre-exemples qui suivent et qui sont donnés à titre illustratif et non limitatif. Dans ces exemples et contre-exemples, les pourcentages sont donnés en poids de constituant par rapport au volume de solvant (W/V), sauf indication contraire.

### EXEMPLES :

### Exemple 1

### Test de stabilité :

La stabilité d'un rétinoïde est définie conformément à la présente invention par le pourcentage du rétinoïde qui est resté sous sa forme d'origine après que la composition le comprenant ait été stockée pendant un temps donné et à une température donnée.

### ESSAIS :

### Test de stabilité du rétinol ail trans à 0.3% dans un mélange eau/éthanol en conservation à 45°C

Les conditions de l'étude sont les suivantes :
- composé à tester à 0.1% (en poids/volume) dans l'eau/éthanol (40/60%)
- Rétinol all trans commercialisé par la société Fluka à 0.3% (en poids/volume) dans l'eau/éthanol (40/60%)
- Milieu aérobie avec 55% de volume d'air, 45% volume d'éthanol/eau
- Conservation à 45°C en flacons ambrés.

La dégradation du rétinol dans ces conditions atteint 100% en 7 jours de conservation à 45°C. Les conditions de conservation auxquelles nous avons soumis les compositions sont particulièrement sévères.

On teste les compositions suivantes :
- le rétinol seul à pH 8 et 11 (formules A et B respectivement),
- le rétinol avec la camosine à pH 8,2 (formule comparative C),
- le rétinol avec le composé 1 à pH 5,5 et 8,2 (formules D et E respectivement),
- le rétinol avec le composé 2 à pH 12 (formule F),
- le rétinol avec le composé 3 à pH 8, 2 et 12 (formules G et H respectivement),
- le rétinol avec le composé 4 à pH 8,2 et 11 (formules I et J respectivement, le composé 4 est le N-(5-amino-1-oxopentyl)-L-histidine).

On suit par HPLC (sur colonne Sphérisorb ODS1 C18, par élution avec un mélange H₂O/CH₃CO₂H/CH₃CO₂NH₄/Acétonitrile : 15/1/0,4/83,6) le taux de rétinol résiduel à 7 (TR1) et 15 jours (TR2) de conservation à 45°C en milieu aérobie (55% de volume d'air).

On obtient les résultats suivants :

| Formule | TR1 | TR2 |
|---|---|---|
| Formule A | 0% | 0% |
| Formule B | 0% | 0% |
| Formule C | 17% | 4% |
| Formule D | 0% | 0% |
| Formule E | 78% | 63% |
| Formule F | 44% | 10% |
| Formule G | 78% | 55% |
| Formule H | 70% | 53% |
| Formule I | 2% | 0% |
| Formule J | 69% | 20% |

Ainsi, on constate que, à pH comparable, la carnosine stabilise beaucoup moins bien le rétinol que les dérivés d'histine utilisés selon l'invention.

### Exemple 2

Des compositions selon l'invention sont réalisées telles que décrites ci-dessous avec les composés 1, 2, 3 et 4 à titre de composés de formule (I).

| Nom CTFA | Formule 1 |
|---|---|
| alcool cétylique | 5% |
| Glyceryl stéarate | 3% |
| P.E.G. 50 stéarate | 3% |
| huile minérale | 18.4% |
| Caprilic/Capric triglycerides | 5% |
| Rétinol ail trans fluka | 0.1% |
| eau | QSP 100% |
| composé de formule (I) | 0.5% |

## Revendications

1. Association **caractérisée par le fait qu'**elle comprend au moins un rétinoïde choisi parmi la vitamine A, le rétinal et les précurseurs bio-convertibles de la vitamine A, et au moins un dérivé d'histidine répondant à la formule générale (I) suivante : 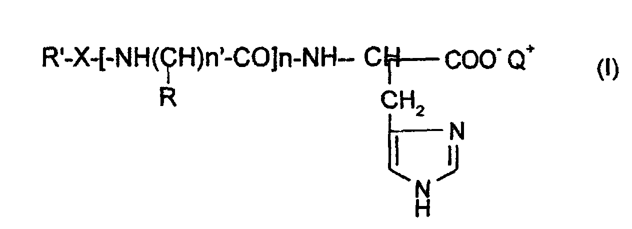 dans laquelle :
n est un entier allant de 0 à 5,
n' est un entier allant de 1 à 16,
R représente une chaîne latérale d'un acide aminé,
X représente un radical choisi parmi les radicaux de formules, lues de gauche à droite : -CO-, -O-CO-, -NH-CO-, -SO2-, -NH-CO-CO-, -O-CO-CO-,
R' représente un radical alkyle linéaire ou ramifié, saturé ou insaturé, éventuellement hydroxylé, en C6 à C22 ou un radical aminoalkyle en C6 à C22, la fonction amine étant événtuellement protégée sous forme d'acétamide ou substitué par un ou deux groupements alkyles inférieurs,
à la condition que R' soit un radical aminoalkyle tel que défini ci-dessus lorsque X = -CO-,
Q⁺ représente H⁺ ou un cation organique ou minéral,
ainsi que les sels d'addition d'un composé de formule (I) avec un acide organique ou minéral.

2. Association selon la revendication 1, **caractérisée par le fait que** le rétinoïde est choisi parmi le rétinol de type all-trans et le rétinol de type 13-cis.

3. Association selon la revendication 1, **caractérisée par le fait que** le rétinoïde est choisi parmi les esters de rétinol en C₁-C₆.

4. Association selon l'une quelconque des revendications précédentes, **caractérisée par le fait que** le pH de l'association est supérieur ou égal à 6, de préférence supérieur ou égal à 7.

5. Association selon l'une quelconque des revendications précédentes, **caractérisée par le fait que** les dérivés d'histidine de formule (I) présentent au moins une des caractéristiques suivantes :
R' désigne un radical alkyle, linéaire ou ramifié, saturé, ayant de 8 à 18 atomes de carbone,
n est un entier de 1 à 5, et
n' est un entier allant de 1 à 11.

6. Association selon l'une quelconque des revendications précédentes, **caractérisée par le fait que** les dérivés d'histidine de formule (I) sont choisis parmi ceux de formules suivantes : 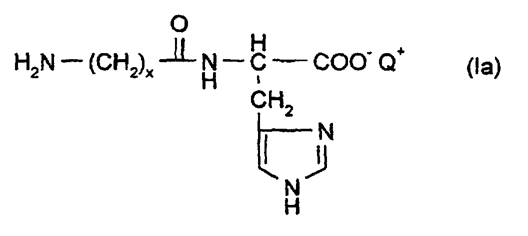 dans laquelle :
x est un entier allant de 6 à 22 et Q⁺ étant tel que décrit dans les revendications précédentes,
et 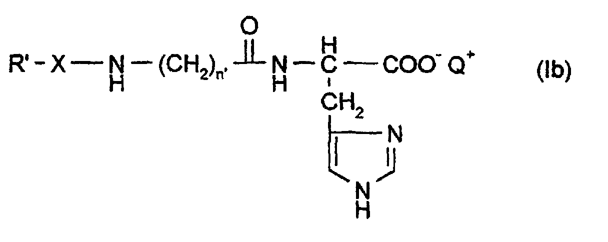 dans laquelle R', X, Q⁺ et n' sont tels que décrits dans les revendications précédentes.

7. Association selon l'une quelconque des revendications précédentes, caractérisée ne ce que les dérivés d'histidine de formule (I) sont choisis parmi :
- la N-octyloxycarbonyl-β-alanyl-L-histidine,
- la N-dodecyloxycarbonyl-β-alanyl-L-histidine,
- la N-(12-amino-1-oxododécyl)-L-histidine,
- le chlorhydrate de N-2-éthylhexyloxycarbonyl-β-alanyl-L-histidine,
- la N-hexadécyloxycarbonyl-β-alanyl-L-histidine,
- la N-octylaminocarbonyl-β-alanyl-L-histidine,
- la N-dodecylaminocarbonyl-β-alanyl-L-histidine,
- la N-dodecylsulfonyl-β-alanyl-L-histidine,
- la N-dodecylamino-oxalyl-β-alanyl-L-histidine.

8. Association selon l'une quelconque des revendications précédentes, **caractérisée par le fait qu'**elle comprend en outre au moins un filtre solaire.

9. Composition cosmétique et/ou dermatologique, **caractérisée par le fait qu'**elle comprend une association selon l'une quelconque des revendications 1 à 8 et un support physiologiquement acceptable.

10. Composition selon la revendication 9, **caractérisée par le fait que** le rétinoïde est présent en une quantité allant de 0,0001 à 10% en poids par rapport au poids total de la composition, avantageusement de 0,001 à 3%, encore plus préférentiellement de 0,01 à 1%.

11. Composition selon l'une quelconque des revendications 9 et 10, **caractérisée par le fait que** le dérivé d'histidine de formule (I) est présent en une quantité allant de 0,001 à 20% en poids par rapport au poids total de la composition, avantageusement de 0,05 à 10%.

12. Composition selon l'une quelconque des revendications 9 à 11, **caractérisée par le fait qu'**elle comprend en outre au moins un composé choisi parmi : les agents chélatant, les polysaccharides, les huiles ayant un indice d'iode supérieur ou égal à 70, les polyéthylène glycol et/ou les polypropylène glycols, les hydroxycarboxylates, les acides aminés et leurs sels, les anti-oxydants tels que le butyl hydroxytoluène, le butyl hydroxyanisole, les α, β, γ, δ-tocophérols, la nordihydrogaiaretine, le gallate de propyle, les esters d'acides gras et de vitamine C, l'acide ascorbique, les sels d'acide ascorbique, l'acide iso-ascorbique, les sels d'acide iso-ascorbique, l'acide sorbique, les sels d'acide sorbique, le butanediol, les esters d'acide gras et de pentaérythritol, les esters gras de triméthylolpropane, les argiles minérales hydrophiles.

13. Composition selon l'une quelconque des revendications 9 à 12, **caractérisée par le fait qu'**elle comprend en outre au moins un agent antiacnéïque spécifique, antiséborrhéique et/ou antibactérien.

14. Composition selon l'une quelconque des revendications 9 à 13, **caractérisée par le fait qu'**elle est sous la forme d'une lotion, d'un gel, d'une émulsion eau-dans-huile, d'une émulsion huile-dans-eau, d'une émulsion triple, de nanocapsules, de liposomes, de nanoémulsions, d'oléosomes.

15. Composition selon l'une quelconque des revendications 9 à 14, **caractérisée par le fait qu'**elle est sous la forme d'une crème de soin, d'un lait, d'un tonique, d'un produit nettoyant et/ou démaquillant, d'un masque, d'un produit de gommage, d'un exfoliant, d'un produit de protection solaire, d'un fond de teint, d'une crème teintée, d'un rouge-à-lèvres.

16. Utilisation d'au moins un dérive d'histidine de formule (I) tel que défini à l'une quelconque des revendications 1 et 5 à 7 pour améliorer la stabilité d'une composition comprenant un rétinoïde choisi parmi la vitamine A, le rétinal et les précurseurs bio-convertibles de la vitamine A.

17. Procédé pour améliorer la stabilité d'une composition comprenant un rétinoïde choisi parmi la vitamine A, le rétinal et les précurseurs bio-convertibles de la vitamine A, ce procédé consistant à associer audit rétinoïde une quantité efficace d'au moins un dérivé d'histidine de formule (I) tel que défini à l'une quelconque des revendications 1 et 5 à 7.

18. Utilisation d'une association selon l'une quelconque des revendications 1 à 8 dans ou pour la préparation d'une composition destinée au traitement dermatologique de lutte contre, et/ou de prévention de, l'irritation, l'inflammation, l'immunosuppression et/ou l'acné.

19. Procédé de traitement cosmétique consistant à lutter contre les signes du vieillissement, plus particulièrement les signes du vieillissement induits par la photoperoxydation, en particulier la photoperoxydation du squalène et/ou du collagène par application topique sur la peau et/ou le cuir chevelu et/ou les cheveux d'une composition selon l'une quelconque des revendications 9 à 15.

20. Utilisation d'une composition cosmétique selon l'une quelconque des revendications 9 à 15 pour lutter contre et/ou prévenir les signes du vieillissement cutané ou des cheveux.

21. Utilisation selon la revendication 20 pour lutter contre et/ou prévenir les signes du vieillissement photoinduits de la peau et/ou des cheveux.

22. Composition dermatologique selon l'une quelconque des revendications 9 à 15 destinée à lutter contre les signes du vieillissement cutané ou des cheveux, particulièrement les signes du vieillissement induits par la photoperoxydation, en particulier la photoperoxydation du squalène et/ou du collagène.

## Patentansprüche

1. Kombination, **dadurch gekennzeichnet, dass** sie mindestens ein Retinoid, das unter Vitamin A, Retinal und biologisch umwandelbaren Vorläufern von Vitamin A ausgewählt ist, und mindestens ein Histidinderivat der folgenden allgemeinen Formel (I) enthält: 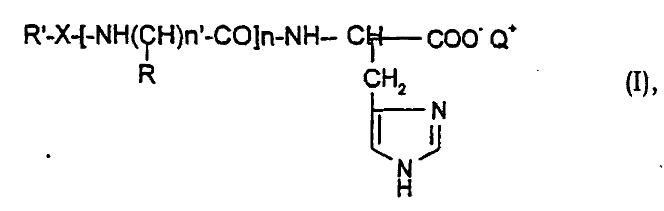 worin bedeuten:
· n 0 oder eine ganze Zahl von 1 bis 5,
· n'eine ganze Zahl von 1 bis 16,
· R eine Seitenkette einer Aminosäure,
· X eine Gruppe, die unter den Gruppen der folgenden Formeln (von links nach rechts zu lesen) ausgewählt ist:
-CO-, -O-CO-, -NH-CO-, -SO₂-, -NH-CO-CO- und -O-CO-CO-,
· R' eine geradkettige oder verzweigte, gesättigte oder ungesättigte, gegebenenfalls hydroxylierte Alkylgruppe mit 6 bis 22 Kohlenstoffatomen oder eine Aminoalkylgruppe mit 6 bis 22 Kohlenstoffatomen, wobei die Aminogruppe gegebenenfalls in Form von Acetamid geschützt oder mit einer oder zwei niederen Alkylgruppen substituiert ist,
mit der Maßgabe, dass R' eine oben definierte Aminoalkylgruppe ist, wenn X = -CO-,
· Q⁺ H⁺ oder ein organisches oder anorganisches Kation,
sowie die Additionssalze einer Verbindung der Formel (I) mit einer organischen oder anorganischen Säure.

2. Kombination nach Anspruch 1, **dadurch gekennzeichnet, dass** das Retinoid unter Retinol vom Typ all-trans oder Retinol vom Typ 13-cis ausgewählt ist.

3. Kombination nach Anspruch 1, **dadurch gekennzeichnet, dass** das Retinoid unter den C₁₋₆-Retinolestern ausgewählt ist.

4. Kombination nach einem der vorhergehenden Ansprüche, **dadurch gekennzeichnet, dass** der pH der Kombination 6 ist oder darüber liegt und vorzugsweise mindestens 7 beträgt.

5. Kombination nach einem der vorhergehenden Ansprüche, **dadurch gekennzeichnet, dass** die Histidinderivate der Formel (I) mindestens eine der folgenden Eigenschaften aufweisen:
R' bedeutet eine gesättigte, geradkettige oder verzweigte Alkylgruppe mit 8 bis 18 Kohlenstoffatomen,
n ist eine ganze Zahl von 1 bis 5, und
n' ist eine ganze Zahl von 1 bis 11.

6. Kombination nach einem der vorhergehenden Ansprüche, **dadurch gekennzeichnet, dass** die Histidinderivate der Formel (I) unter den Verbindungen der folgenden Formeln ausgewählt sind: 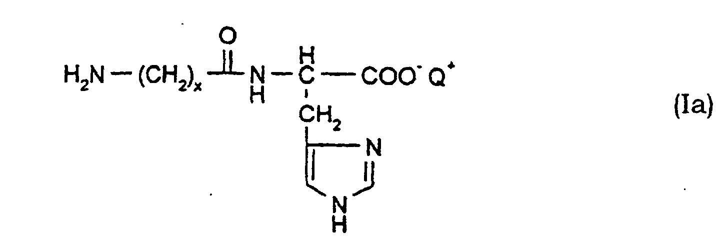 worin bedeuten:
x eine ganze Zahl von 6 bis 22 und Q⁺ die in den vorhergehenden Ansprüchen angegebenen Bedeutungen,
und 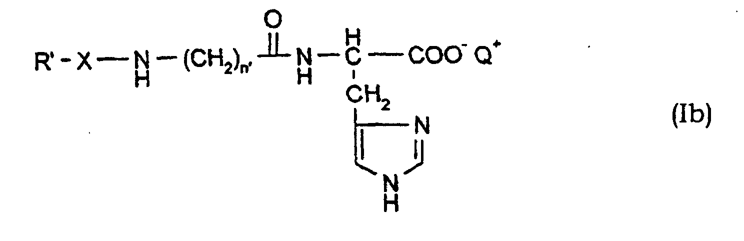 worin bedeuten:
R', X, Q⁺ und n' die in den vorhergehenden Ansprüchen angegebenen Bedeutungen.

7. Kombination nach einem der vorhergehenden Ansprüche, **dadurch gekennzeichnet, dass** die Histidinderivate der Formel (I) ausgewählt sind unter:
- N-Octyloxycarbonyl-β-alanyl-L-histidin,
- N-Dodecyloxycarbonyl-β-alanyl-L-histidin,
- N-(12-Amino-1-oxo-dodecyl)-L-histidin,
- N-2-Ethylhexyloxycarbonyl-β-alanyl-L-histidin-Hydrochlorid,
- N-Hexadecyloxycarbonyl-β-alanyl-L-histidin,
- N-Octylaminocarbonyl-β-alanyl-L-histidin,
- N-Dodecylaminocarbonyl-β-alanyl-L-histidin,
- N-Dodecylsulfonyl-β-alanyl-L-histidin, und
- N-Dodecylamino-oxalyl-β-alanyl-L-histidin.

8. Kombination nach einem der vorhergehenden Ansprüche, **dadurch gekennzeichnet, dass** sie ferner mindestens ein Sonnenschutzfilter enthält.

9. Kosmetische und/oder dermatologische Zusammensetzung, **dadurch gekennzeichnet, dass** sie eine Kombination nach einem der Ansprüche 1 bis 8 in einem physiologisch akzeptablen Träger enthält.

10. Zusammensetzung nach Anspruch 9, **dadurch gekennzeichnet, dass** das Retinoid in Mengenanteilen im Bereich von 0,0001 bis 10 Gew.-%, vorteilhaft 0,001 bis 3 Gew.-% und noch bevorzugter 0,01 bis 1 Gew.-%, bezogen auf das Gesamtgewicht der Zusammensetzung, vorliegt.

11. Zusammensetzung nach einem der Ansprüche 9 und 10, **dadurch gekennzeichnet, dass** das Histidinderivat der Formel (I) in Mengenanteilen im Bereich von 0,001 bis 20 Gew.-% und vorteilhaft 0,05 bis 10 Gew.-%, bezogen auf das Gesamtgewicht der Zusammensetzung, vorliegt.

12. Zusammensetzung nach einem der Ansprüche 9 bis 11, **dadurch gekennzeichnet, dass** sie ferner mindestens eine Verbindung enthält, die ausgewählt ist unter: Chelatbildnern, Polysacchariden, Ölen, die einen Iodindex von mindestens 70 aufweisen, Polyethylenglykol und/oder Polypropylenglykolen, Hydroxycarboxylaten, Aminosäuren und ihren Salzen, Antioxidantien, wie Butylhydroxytoluol, Butylhydroxyanisol, α-, β-, γ- und δ-Tocopherol, Nordihydrogaiaretin, Propylgallat, Estern von Fettsäuren und Vitamin C, Ascorbinsäure, Salzen von Ascorbinsäure, Isoascorbinsäure, Salzen von Isoascorbinsäure, Sorbinsäure, Salzen von Sorbinsäure, Butandiol, Estern von Fettsäuren und Pentaerythrit, Fettsäurestern von Trimethylolpropan und hydrophilen anorganischen Tonen.

13. Zusammensetzung nach einem der Ansprüche 9 bis 12, **dadurch gekennzeichnet, dass** sie ferner mindestens ein spezielles Mittel gegen Akne, gegen Seborrhoe und/oder gegen Bakterien enthält.

14. Zusammensetzung nach einem der Ansprüche 9 bis 13, **dadurch gekennzeichnet, dass** sie als Lotion, Gel, Wasser-in-Öl-Emulsion, Öl-in-Wasser-Emulsion, dreifache Emulsion, Nanokapseln, Liposomen, Nanoemulsionen oder Oleosomen vorliegt.

15. Zusammensetzung nach einem der Ansprüche 9 bis 14, **dadurch gekennzeichnet, dass** sie als Creme zur Pflege, Milch, Tonikum, Produkt zum Reinigen und/oder Abschminken, Maske, abradierendes Produkt, Exfoliationsmittel, Produkt zum Sonnenschutz, Makeup, getönte Creme oder Lippenstift vorliegt.

16. Verwendung mindestens eines Histidinderivats der Formel (I) nach einem der Ansprüche 1 und 5 bis 7, um die Stabilität einer Zusammensetzung zu verbessern, die ein Retinoid enthält, das unter Vitamin A, Retinal und biologisch umwandelbaren Precursoren von Vitamin A ausgewählt ist.

17. Verfahren zur Verbesserung der Stabilität einer Zusammensetzung, die ein Retinoid enthält, das unter Vitamin A, Retinal und biologisch umwandelbaren Precursoren von Vitamin A ausgewählt ist, wobei das Verfahren darin besteht, eine wirksame Menge mindestens eines Histidinderivats der Formel (I) nach einem der Ansprüche 1 und 5 bis 7 mit dem Retinoid zu kombinieren.

18. Verwendung einer Kombination nach einem der Ansprüche 1 bis 8 in einer Zusammensetzung oder zur Herstellung einer Zusammensetzung, die zur dermatologischen Bekämpfung und/oder Vorbeugung von Reizungen, Entzündungen, Immunosuppression und/oder Akne vorgesehen ist.

19. Verfahren zur kosmetischen Behandlung, das darin besteht, die Anzeichen der Alterung, insbesondere die durch Photoperoxidation und ganz besonders die durch Photoperoxidation von Squalen und/oder Kollagen hervorgerufenen Anzeichen der Alterung zu bekämpfen, indem auf die Haut und/oder die Kopfhaut und/oder die Haare topisch eine Zusammensetzung nach einem der Ansprüche 9 bis 15 aufgebracht wird.

20. Verwendung einer kosmetischen Zusammensetzung nach einem der Ansprüche 9 bis 15, um die Anzeichen der Alterung der Haut oder der Haare zu bekämpfen und/oder ihnen vorzubeugen.

21. Verwendung nach Anspruch 20, um die durch Licht hervorgerufenen Anzeichen der Alterung der Haut und/oder der Haare zu bekämpfen und/oder ihnen vorzubeugen.

22. Dermatologische Zusammensetzung nach einem der Ansprüche 9 bis 15, die dazu vorgesehen ist, die Anzeichen der Alterung der Haut oder der Haare, insbesondere die durch Photoperoxidation und ganz besonders die durch Photoperoxidation von Squalen und/oder Kollagen hervorgerufenen Anzeichen der Alterung zu bekämpfen.

## Claims

1. Combination, **characterized in that** it comprises at least one retinoid chosen from vitamin A, retinal and bioconvertible precursors of vitamin A, and at least one histidine derivative corresponding to the general formula (I) below: 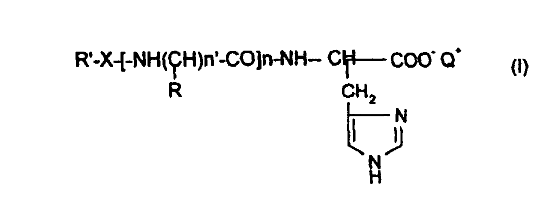 in which:
n is an integer ranging from 0 to 5,
n' is an integer ranging from 1 to 16,
R represents a side chain of an amino acid,
X represents a radical chosen from the radicals of formulae, read from left to right:
-CO-, -O-CO-, -NH-CO-, -SO2-, -NH-CO-CO-, -O-CO-CO-,
R' represents a linear or branched, saturated or unsaturated, optionally hydroxylated, C6 to C22 alkyl radical or C6 to C22 aminoalkyl radical, the amine function optionally being protected in the form of acetamide or substituted with one or two lower alkyl groups,
with the proviso that R' is an aminoalkyl radical as defined above when X = -CO-
Q⁺ represents H⁺ or an organic or inorganic cation,
as well as the addition salts of a compound of formula (I) with an organic or inorganic acid.

2. Combination according to Claim 1, **characterized in that** the retinoid is chosen from retinol of all-trans type and retinol of 13-cis type.

3. Combination according to Claim 1, **characterized in that** the retinoid is chosen from C₁-C₆ retinol esters.

4. Combination according to any one of the preceding claims, **characterized in that** the pH of the combination is greater than or equal to 6, preferably greater than or equal to 7.

5. Combination according to any one of the preceding claims, **characterized in that** the histidine derivatives of formula (I) have at least one of the following characteristics:
R' denotes a linear or branched, saturated alkyl radical containing from 8 to 18 carbon atoms,
n is an integer from 1 to 5, and
n' is an integer ranging from 1 to 11.

6. Combination according to any one of the preceding claims, **characterized in that** the histidine derivatives of formula (I) are chosen from those of the following formulae: 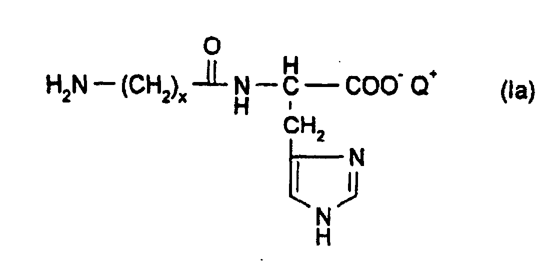 in which:
x is an integer ranging from 6 to 22 and Q⁺ being as described in the preceding claims,
and 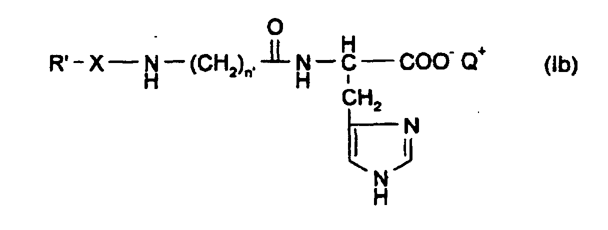 in which R', X, Q⁺ and n' are as described in the preceding claims.

7. Combination according to any one of the preceding claims, **characterized in that** the histidine derivatives of formula (I) are chosen from:
- N-octyloxycarbonyl-β-alanyl-L-histidine,
- N-dodecyloxycarbonyl-β-alanyl-L-histidine,
- N-(12-amino-1-oxododecyl)-L-histidine,
- N-2-ethylhexyloxycarbonyl-β-alanyl-L-histidine hydrochloride,
- N-hexadecyloxycarbonyl-β-alanyl-L-histidine,
- N-octylaminocarbonyl-β-alanyl-L-histidine,
- N-dodecylaminocarbonyl-β-alanyl-L-histidine,
- N-dodecylsulphonyl-β-alanyl-L-histidine,
- N-dodecylamino-oxalyl-β-alanyl-L-histidine.

8. Combination according to any one of the preceding claims, **characterized in that** it also comprises at least one sunscreen.

9. Cosmetic and/or dermatological composition, **characterized in that** it comprises a combination according to any one of Claims 1 to 8 and a physiologically acceptable support.

10. Composition according to Claim 9, **characterized in that** the retinoid is present in an amount ranging from 0.0001 to 10% by weight relative to the total weight of the composition, advantageously from 0.001 to 3% and even more preferably from 0.01 to 1%.

11. Composition according to either of Claims 9 and 10, **characterized in that** the histidine derivative of formula (I) is present in an amount ranging from 0.001 to 20% by weight relative to the total weight of the composition, advantageously from 0.05 to 10%.

12. Composition according to any one of Claims 9 to 11, **characterized in that** it also comprises at least one compound chosen from: chelating agents, polysaccharides, oils with an iodine number of greater than or equal to 70, polyethylene glycols and/or polypropylene glycols, hydroxycarboxylates, amino acids and their salts, antioxidants such as butyl hydroxytoluene, butyl hydroxyanisole, α, β, γ and δ-tocopherols, nordihydrogaiaretine, propyl gallate, fatty acid esters of vitamin C, ascorbic acid, ascorbic acid salts, isoascorbic acid, isoascorbic acid salts, sorbic acid, sorbic acid salts, butanediol, fatty acid esters of pentaerythritol, fatty esters of trimethylolpropane and hydrophilic mineral clays.

13. Composition according to any one of Claims 9 to 12, **characterized in that** it also comprises at least one specific antiacne, antiseborrhoeic and/or antibacterial agent.

14. Composition according to any one of Claims 9 to 13, **characterized in that** it is in the form of a lotion, a gel, a water-in-oil emulsion, an oil-in-water emulsion, a triple emulsion, nanocapsules, liposomes, nanoemulsions or oleosomes.

15. Composition according to any one of Claims 9 to 14, **characterized in that** it is in the form of a care cream, a milk, a tonic, a cleansing and/or make-up-removing product, a mask, an erasing product, an exfoliant, a sunscreen product, a foundation, a tinted cream or a lipstick.

16. Use of at least one histidine derivative of formula (I) as defined in any one of Claims 1 and 5 to 7 for improving the stability of a composition comprising a retinoid chosen from vitamin A, retinal and bioconvertible precursors of vitamin A.

17. Process for improving the stability of a composition comprising a retinoid chosen from vitamin A, retinal and bioconvertible precursors of vitamin A, this process consisting in combining an effective amount of at least one histidine derivative of formula (I) as defined in any one of Claims 1 and 5 to 7 with the said retinoid.

18. Use of a combination according to any one of Claims 1 to 8, in, or for the preparation of, a composition intended for dermatological treatment for controlling and/or preventing irritation, inflammation, immunosuppression and/or acne.

19. Cosmetic treatment process which consists in controlling the signs of ageing, more particularly the signs of ageing induced by photoperoxidation, in particular the photoperoxidation of squalene and/or collagen, by topical application to the skin and/or the scalp and/or the hair of a composition according to any one of Claims 9 to 15.

20. Use of a cosmetic composition according to any one of Claims 9 to 15, for controlling and/or preventing the signs of ageing of the skin or the hair.

21. Use according to Claim 20, for controlling and/or preventing the signs of light-induced ageing of the skin and/or the hair.

22. Dermatological composition according to any one of Claims 9 to 15, which is intended for controlling the signs of ageing of the skin or the hair, particularly the signs of ageing induced by photoperoxidation, in particular the photoperoxidation of squalene and/or collagen.
